# EUROPEAN PATENT SPECIFICATION

(11) **EP 3 964 246 B1**
(45) Date of publication and mention of the grant of the patent: **14.05.2025**
(21) Application number: 20194067.3
(22) Date of filing: 02.09.2020
(51) Int. Cl.: A61M 1/36, A61M 1/30

(54) **METHOD FOR PRIMING AN EXTRACORPOREAL BLOOD CIRCUIT AND DEVICES**
VERFAHREN ZUM FÜLLEN EINES EXTRAKORPORALEN BLUTKREISLAUFS UND VORRICHTUNGEN
PROCÉDÉ D'AMORÇAGE D'UN CIRCUIT SANGUIN EXTRACORPOREL ET DISPOSITIFS

(43) Date of publication of application: 09.03.2022
(73) Proprietor: Fresenius Medical Care Deutschland GmbH, 61352 Bad Homburg (DE)
(72) Inventor: Canaud, Bernard, 34090 Montpellier (FR)
(74) Representative: Bobbert & Partner Patentanwälte PartmbB

(56) References cited:
- US-A1- 2017 021 086
- US-A1- 2017 304 522

## Description

The present invention relates to a method according to claim 1. It also relates to a control device or closed-loop control device according to claim 6 and a blood treatment apparatus according to claim 7. Furthermore the present invention relates to a digital storage medium according to claim 10, a computer program product according to claim 11 as well as a computer program according to claim 12.

Extracorporeal blood treatment using dialysis is known from practice such as in US 2017 021086. Whereby the patient's blood is withdrawn and extracorporeally fed along a blood circuit and through, e.g. a blood filter. The blood filter comprises a blood chamber through which blood is guided, and a dialysis liquid chamber, through which dialysis liquid is guided. Both chambers are separated from each other by a semi-permeable membrane. Blood and dialysis liquid are mostly guided through the blood filter by the counter-current principle. The blood is purified in the blood filter, on exiting the blood filter the dialysis liquid, from now on referred to as effluate or effluent, is regarded as used and is discarded. In addition to the dialysate, the effluent to be discarded also comprises filtrate (or ultra-filtrate), which comprises water that has been withdrawn from the blood in the blood filter. Filtrate and dialysate will be referred to individually or collectively in the following simply as effluent. In addition to acute cases, dialysis is mainly used with patients who have end-stage renal failure.

Typically before a dialysis patient is connected to the dialysis equipment the extracorporeal circuit has to be primed. The purpose of priming the circuit is to remove air from the blood lines and the dialyzer as well as to remove possible fragments of remaining sterilising agents or other residuals from the disposables elements, such as blood lines and dialyzers that form the extracorporeal circuit, before the patient is connected.

An object of the present invention is to recommend a method for priming an extracorporeal blood circuit before the blood treatment session starts.

Furthermore, a control device or closed-loop control device, a blood treatment apparatus, a suitable digital storage medium, a suitable computer program product and a suitable computer program are to be specified.

The object according to the present invention may be achieved with the method having the features of claim 1, a control device or closed-loop control device having the features of claim 6 and a blood treatment apparatus having the features of claim 7. Further, the object according to the present invention may be achieved with a digital storage medium, a computer program product as well as a computer program according to claims 10, 11 and 12.

The method according to the present invention relates to priming an extracorporeal blood circuit using or providing a blood treatment apparatus comprising a dialysis liquid preparation system. The dialysis liquid preparation system comprises or consists of a source of water, a source of bicarbonate concentrate (e.g., one container, bag or the like) and a source of acid concentrate (e.g., one container, bag or the like), the latter including sodium chloride, an acid, for example citric or acetic acid and further electrolytes, like magnesium chloride and or calcium chloride. The dialysis liquid preparation system does not use a source of sodium chloride only. The dialysis liquid preparation system is configured to prepare a dialysis liquid from those three sources mentioned (water, said bicarbonate concentrate source and said acid concentrate source) only.

The extracorporeal blood circuit comprises an arterial line section, connectable to a patient. It serves to withdraw blood from the patient. Further, the extracorporeal blood circuit comprises a venous line section which is connectable to the patient for returning the blood to her. Moreover, a blood side compartment or blood chamber of a blood filter is comprised by the extracorporeal blood circuit.

The method according to the present invention encompasses the steps of a first phase as follows:
a) preparing a priming solution from said source of water and said source of acid concentrate only to obtain an acid/water solution, wherein bicarbonate from the source of bicarbonate concentrate is absent from said solution;
b) connecting the arterial line section to an outlet of the dialysis liquid preparation system of the blood treatment apparatus, e.g. to the substitution port or another port of the blood treatment apparatus; and
c) filling the extracorporeal blood circuit with said priming solution.

The present invention further relates to a control device or closed-loop control device. It is configured or programmed to carry out and/or to initiate the method according to the present invention or its mechanical steps, in particular in each of the embodiments described herein and in each possible combination of the herein disclosed features, in particular method steps.

The control device or closed-loop control device may comprise devices or may be connected in signal communication with such devices, which can execute the individual method steps or method features which are disclosed herein - and particularly in the claims - and are designed, configured and/or programmed accordingly for this purpose.

The present invention further relates to a blood treatment apparatus for the extracorporeal treatment of a patient's blood which comprises a control device or closed-loop control device according to the present invention.

A digital, in particular non-volatile storage medium, according to the present invention, particularly in the form of a machine readable carrier, particularly in the form of a diskette, storage card, CD, DVD Blu-ray disc or (E)EPROM, FRAM (Ferroelectric RAM) or SSD (Solid-State-Drive) with electronically readable control signals, may be configured to interact with a programmable control device or closed-loop control device in such a way that the, particularly mechanical, steps of the method according to the present invention are initiated or executed.

Thereby, all, any or several of the steps, in particular mechanical steps, of the method according to the present invention may be initiated or executed.

Alternatively or additionally, the digital storage medium according to the present invention may be configured so that a conventional control device or closed-loop control device can be reprogrammed into a control device or closed-loop control device according to the present invention.

A computer program product according to the present invention comprises a transient, volatile program code or a program code saved on a machine readable carrier for initiating or executing the, particularly mechanical, steps of the method according to the present invention, when the computer program product is running on a control device or closed-loop control device.

The computer program product according to the present invention can be understood, for example, as a computer program stored on a carrier, an embedded system being a comprehensive system with a computer program (e.g., electronic device with a computer program), a network of computer-implemented computer programs (e.g. client/server-system, a cloud computing system etc.), or a computer on which a computer program is loaded, run, stored, executed or developed.

Alternatively or additionally hereto, the computer program product according to the present invention may be configured to reprogram a conventional control device or closed-loop control device into a control device or closed-loop control device according to the present invention.

The term "machine-readable carrier" as it is used herein, refers in certain embodiments of the present invention to a carrier, which contains data or information interpretable by software and/or hardware. The carrier may be a data carrier, such as a diskette, a CD, DVD, a USB stick, a flashcard, an SD card or the like, as well as any other storage referred to herein or any other storage medium referred to herein.

A computer program according to the present invention comprises a program code to initiate or execute the steps of the method according to the present invention, particularly the mechanical steps, when the computer program is running on a control device or closed-loop control device. According to the present invention, a computer program can be understood to mean, for example, a physical, ready-for-distribution software product that comprises a program.

Alternatively or additionally, the computer program according to the present invention, may be configured in order to reprogram a conventional control device or closed-loop control device into a control device or closed-loop control device according to the present invention.

It also applies to the computer program product according to the present invention and the computer program according to the present invention, that all, any, or several of the steps, in particular mechanical steps, of the method according to the present invention may be initiated or executed.

Embodiments according to the present invention may comprise one or several of the aforementioned or following features in any combination, unless the person skilled in the art recognizes their combination as technically impossible. Embodiments according to the present invention are further subject-matter of the dependent claims.

In all of the following statements, the use of the expression "may be" or "may have" and so on, is to be understood synonymously with "preferably is" or "preferably has," and so on respectively, and is intended to illustrate an embodiment according to the present invention.

Whenever numerical words are mentioned herein, the person skilled in the art shall recognize or understand them as indications of a numerical lower limit. Unless it leads the person skilled in the art to an evident contradiction, the person skilled in the art shall comprehend the specification for example of "one" as encompassing "at least one". This understanding is also equally encompassed by the present invention as the interpretation that a numeric word, for example, "one" may alternatively mean "exactly one", wherever this is evidently technically possible for the person skilled in the art. Both are encompassed by the present invention and apply herein to all numerical words used.

When it is disclosed herein that the subject-matter according to the present invention comprises one or several features in a certain embodiment, it is also respectively disclosed herein that the subject-matter according to the present invention does, in other embodiments, likewise according to the present invention, explicitly not comprise this or these features, for example, in the sense of a disclaimer. Therefore, for every embodiment mentioned herein it applies that the converse embodiment, e.g. formulated as negation, is also disclosed.

Whenever an embodiment is mentioned herein, it is then an exemplary embodiment according to the present invention.

In some embodiments, the temperature of the priming solution is kept below 35°C, preferably between 25°C and 30°C during the first phase of the method according to the present invention.

In several embodiments, the method according to the present invention further encompasses adding bicarbonate from the source of bicarbonate concentrate to the priming solution in a second phase following the first phase.

In some embodiments of the method, the temperature of the priming solution is raised to more than 30°C, preferably more than 35°C, for example to 36°C to 37°C, during the second phase.

In several embodiments of the method, the priming solution prepared in the first phase and/or prepared in the second phase is being circulated within the extracorporeal circuit for at least 5 minutes before the priming is considered as completed.

In some embodiments, the blood treatment apparatus according to the present invention is embodied as a hemodialysis apparatus, hemofiltration apparatus or hemodiafiltration apparatus. In particular, it is embodied as an apparatus for acute or chronic renal replacement therapy.

In several embodiments, the blood treatment apparatus comprises sensors, arranged and configured in order to measure the sodium concentration of the priming solution.

In some embodiments, the blood treatment apparatus according to the present invention comprises sensors inserted upstream and/or downstream of the dialyzer of the blood treatment apparatus to measure the electrolyte and/or fluid balance, e.g. on the dialysis liquid side or machine side and/or on the blood side. They can be used to determine the sodium concentration as discussed herein.

In some embodiments, the control device or closed-loop control device according to the present invention may be programmed and/or configured in order to control or regulate the blood treatment device in addition to carrying out or causing or initiating the method according to the present invention in cooperation with other devices.

Whenever a suitability or a method step is mentioned herein, the present invention also comprises corresponding programming or a configuration of a suitable device according to the present invention or a section thereof.

Some or all of the embodiments of the present invention may have one, several or all of the advantages listed above and/or below.

By using the method according to the present invention, air is removed from the blood lines and the blood chamber of the dialyzer of the extracorporeal blood circuit. Also, possible fragments of remaining sterilising agents or other residuals are removed from the disposable elements, such as blood lines and dialyzers that form the extracorporeal circuit, before the patient is connected.

In the past, saline solutions from bags of physiological saline solution were used to prime the extracorporeal blood circuit. Later, dialysis equipment was available that could prepare the substitution fluid for hemofiltration or hemodiafiltration on-line while obtaining a sterile and pyrogen-free fluid. Such on-line prepared substitution fluid has been also used for priming, which also is cost saving and convenient from a handling point of view.

However, due to drawbacks caused by using substitution fluid for priming, after which the patients often experienced problems, e.g. not feeling well, clinics have often gone back to priming with saline from bags after having experienced such problems.

For using remarkably less bicarbonate with the priming solution prepared according to the present invention, a priming solution could be suggested by the present disclosure.

Advantageously, with the present invention an almost bubble-free rinsing solution, which applies to both bicarbonate and CO₂ bubbles, may be provided and used before starting a dialysis treatment. Hence formation of microbubbles that could remain in the extracorporeal blood circuit even beyond the priming procedure is minimised or avoided. As a result, it is less likely that microbubbles will be present in the extracorporeal blood circuit once the priming process has been completed. This may contribute to the patient's safety and may help to reduce nursing preparation time. In particular, typical nursing steps such as setting up the tubing, dialyzer, or the blood treatment apparatus altogether, and degassing of the same may be facilitated, and time needed for such steps may be significantly reduced.

A further advantage of the present invention may be seen in the two-step use of the on-line produced bicarbonate dialysis liquid. It may be firstly used as an acidic liquid for priming and maintaining the extracorporeal blood circuit and secondly as a regular bicarbonate dialysis liquid during a dialysis session.

As an acid solution is used for priming the extracorporeal blood circuit, an additional interpatient disinfection thereof may be omitted. This may help to save time and money.

Following the steps of the method according to the present invention, less bicarbonate may be needed which is of benefit both for costs and logistics.

When using the present invention, calcium/magnesium carbonate precipitation may advantageously be prevented. A calcification of the hydraulic circuit may, therefore, be advantageously avoided.

Also, the risk of micro-bacterial or micro-organism contamination and/or bacterial growth within the extracorporeal circuit may be advantageously prevented. This may contribute to the patient's safety.

Providing and monitoring the conductivity cell as disclosed herein may further contribute to the patient's safety.

A further advantage of the present invention is that it is easy to implement.

All advantages achievable with the method according to the present invention can also be achieved undiminished with the devices according to the present invention, and vice versa.

The present invention is exemplarily explained with regard to the accompanying drawings in which same reference numerals refer to the same or similar components. In the figures the following applies:
- **Fig. 1**: shows in a simplified, schematic representation a blood treatment apparatus according to the present invention with an extracorporeal blood circuit in a first embodiment or the representation of a flow diagram of a blood treatment apparatus according to the present invention, exemplarily embodied as a hemodiafiltration apparatus;
- **Fig. 2**: shows a schematic representation of an exemplary implementation of the first phase of the method according to the present invention; and
- **Fig. 3**: shows a schematic representation of an exemplary implementation of the second phase of the method according to the present invention.

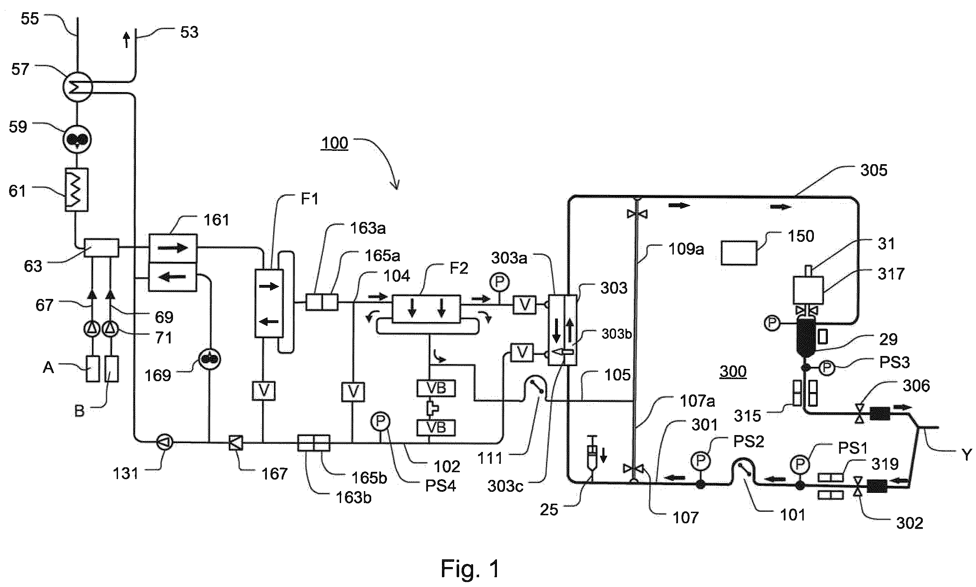

**Fig. 1** shows an extracorporeal blood circuit 300, which can be connected to the vascular system of the patient (not shown) for a treatment via double-needle access, or via single-needle access, using, for example, an additional Y-connector (reference numeral Y) as shown in Fig. 1. The blood circuit 300 can optionally be present in sections thereof in or on a blood cassette.

Pumps, actuators and/or valves in the area of the blood circuit 300 are connected to a blood treatment apparatus 100 according to the present invention or for example, to a control device 150 comprised within.

The blood circuit 300 comprises (or is connected to) an arterial patient tubing clamp 302 and an arterial connection needle of an arterial section or of an arterial patient line, a blood withdrawal line or a first line 301. The blood circuit 300 further comprises (or is connected to) a venous patient tubing clamp 306 and a venous connection needle of a venous section, a venous patient line, blood return line or second line 305.

A blood pump 101 is provided in or on the first line 301, a substituate pump 111 is connected to a dialysis liquid inlet line 104 for conveying fresh dialysis liquid, which is filtered through a further filter (F2) (substituate). A substituate line 105 can be in fluid communication with the inlet line 104. Using the substituate pump 111, substituate can be introduced by pre-dilution via a pre-dilution valve 107, or by post-dilution via a post-dilution valve 109, into line sections via corresponding lines 107a or 109a, for example into the arterial line section 301 or into the venous line section 305 (here between a blood chamber 303b of a blood filter 303 and a venous air separation chamber or venous blood chamber 29) of the blood circuit 300.

The blood filter 303 comprises the blood chamber 303b which is connected to the arterial line section 301 and the venous line section 305. A dialysis liquid chamber 303a of the blood filter 303 is connected to the dialysis liquid inlet line 104 leading to the dialysis liquid chamber 303a and a dialysate outlet line 102 leading away from the dialysis liquid chamber 303a which guides dialysate, i.e. used dialysis liquid. Dialysis liquid chamber 303a and blood chamber 303b are separated by a mostly semi-permeable membrane 303c. This membrane is what separates the blood side with the extracorporeal blood circuit 300 and the machine side with the dialysis liquid circuit or dialysate circuit, which is shown in Fig. 1 to the left of the membrane 303c.

The arrangement in Fig. 1 encompasses an optional detector 315 for detecting air and/or blood. The arrangement in Fig. 1 further encompasses one or two pressure sensors PS1 (upstream of the blood pump 101) and PS2 (downstream of the blood pump 101, which measures the pressure upstream of the blood filter 303 ("pre-hemofilter")) at the positions shown in Fig. 1. Further pressure sensors may be provided, e.g. the pressure sensor PS3 downstream of the venous blood chamber 29.

An optional single-needle chamber 317 is used in Fig. 1 as a buffer and/or a compensating reservoir in a single-needle procedure in which the patient is connected to the extracorporeal blood circuit 300 via only one of the two blood lines 301, 305.

The arrangement in Fig. 1 additionally comprises an optional detector 319 for detecting air bubbles and/or blood.

An addition point 25 for Heparin may be optionally provided. Shown on the left in Fig. 1 is a mixing apparatus 63 being a dialysis liquid preparation system which, from containers A (for A concentrate via the concentrate supply 67) and B (for B concentrate via the concentrate supply 69), provides a predetermined mixture for the respective solution for use by the blood treatment apparatus 100.

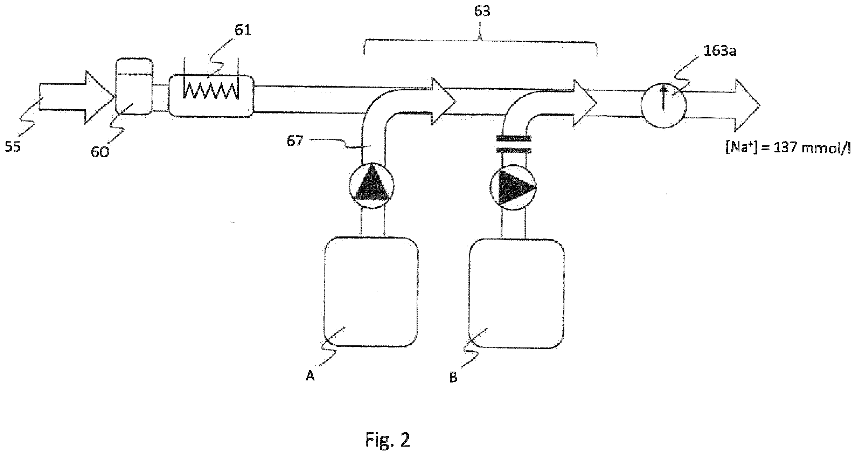

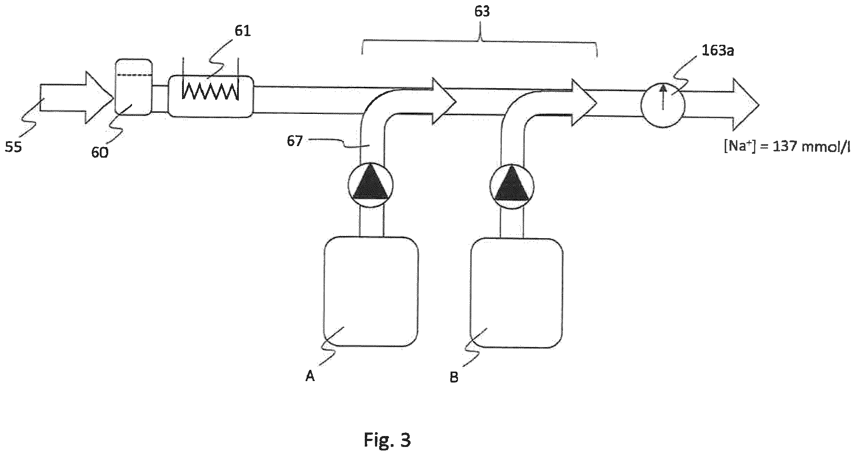

Alternatively, the mixing apparatus 63 as such can be omitted, and the two concentrates can be delivered successively into any fluid line and may come into contact with each other in any component of the blood treatment apparatus 100. After having been brought together in a common vessel (line, tubing, chamber, and so on), the so generated solution may be optionally thoroughly mixed or stirred, e. g., in a device 161 for balancing (e.g., a balancing chamber) as set forth below. Hence, the mixing apparatus 63 as mentioned herein is not restricted to a chamber as shown in Fig. 1. Rather, it may be any other vessel such as the fluid line denoted with 63 in Fig. 2 or Fig. 3.

The solution may contain, in the heater 61 for example, warmed water from the water source 55 (on-line, e.g. as reverse osmosis water or from bags).

A pump 71, that may be referred to as a concentrate pump or sodium pump is in fluid communication with the mixing apparatus 63 and with a source having sodium, such as the container B, and/or conveys therefrom.

Furthermore, an outlet 53 for the effluent can be seen in Fig. 1. An optional heat exchanger 57 and a first flow pump 59, which is suitable for de-gassing, may complete the arrangement shown.

A further pressure sensor for measuring the filtrate pressure or the membrane pressure of the blood filter 303 may be provided as PS4 downstream of the blood filter 303 on the water-side, however preferably upstream of the ultrafiltration pump 131 in the dialysate outlet line 102. Further optional pressure measuring points P may also be provided.

Blood, which leaves the blood filter 303, passes through an optional venous blood chamber 29, which can comprise a de-aeration device 31 and/or may be in fluid communication with a further pressure sensor PS3.

The exemplary arrangement shown in Fig. 1 comprises a control device or closed-loop control device 150. This can be in wired or wireless signal communication with any of the components referred to herein - in particular or especially to the blood pump 101 - in order to control or regulate the blood treatment apparatus 100. It is optionally configured in order to carry out the method described herein, particularly automatically.

By using the device for on-line mixing of the dialysis liquid, a variation in the sodium content thereof controlled by the control device 150, is possible within certain limits. For this purpose, measurements determined via the conductivity sensors 163a, 163b may particularly be taken into account. Should an adjustment of the sodium content of the dialysis liquid (sodium concentration) or of the substituate be required or desired, this can be done by adjusting the delivery rate of the sodium pump 71.

Furthermore, the blood treatment apparatus 100 comprises means for conveying fresh dialysis liquid as well as dialysate. For this purpose, the first flow pump 59, which conveys fresh dialysis liquid towards the blood filter 303, is provided upstream of the blood filter 303. A first valve may be provided between the first flow pump 59 and the blood filter 303, which opens or closes the inlet to the blood filter 303 on the inlet side. A second optional flow pump 169 is provided, for example downstream of the blood filter 303, which conveys dialysate to the outlet 53. A second valve may be provided between the blood filter 303 and the second flow pump 169, which opens or closes the outlet on the outlet side.

Furthermore, the blood treatment apparatus 100 optionally comprises a device 161 for balancing the flow going into and coming out of the dialyzer 303 on the machine side. The device 161 for balancing is preferably arranged in a line section between the first flow pump 59 and the second flow pump 169.

The blood treatment apparatus 100 further encompasses means, such as the ultrafiltration pump 131, for the precise removal of a fluid volume from the balanced circuit as specified by the user and/or by the control device 150.

Sensors such as the optional conductivity sensors 163a, 163b serve to determine the conductivity, which in some embodiments is temperature-compensated, as well as the liquid flow upstream and downstream of the dialyzer 303.

Temperature sensors 165a, 165b can be provided individually or in groups. Temperature readings supplied by them can be used according to the present invention to determine a temperature-compensated conductivity.

A leakage sensor 167 is optionally provided.

Further flow pumps, in addition or as an alternative to, the one indicated with the reference numeral 169 for example, can also be provided.

A row of optional valves is each indicated in Fig. 1 with a V. Bypass valves are indicated with a VB.

In some embodiments the control device 150 determines the electrolyte and/or liquid balancing based on the measurement readings of the afore-mentioned, optional sensors.

Filters F1 and F2 may be provided in series-connection.

The filter F1 here serves exemplarily, via the mixing apparatus 63, to produce sufficiently pure dialysis liquid, even when using impure water, which then flows through the blood filter 303, e.g. according to the counter-current principle.

Exemplarily, here the filter F2 serves to generate a sterile or sufficiently filtered substituate from the sufficiently pure dialysis liquid, which leaves the first filter F1, by filtering out pyrogenic substances, for example. This substituate can safely be added to the patient's blood flowing extracorporeally and thus ultimately be supplied to the patient's body.

The blood treatment apparatus 100 according to the present invention is shown in Fig. 1 as a device for hemo(dia)filtration. However, hemodialysis devices also fall within the scope of the present invention, even though they are not specifically shown in the figures.

The present invention is not limited to the embodiment described above, this serves only as an illustration.

The arrows shown in Fig. 1 generally indicate the direction of flow in Fig. 1.

Fig. 2 shows a schematic representation of an exemplary implementation of the first phase of the method according to the present invention. The components shown are all part of the hydraulic unit of the blood treatment apparatus 100 according to the present invention. In particular, Fig. 1 shows the mixing apparatus 63 used for preparing the priming liquid. As stated before, the mixing apparatus 63 is not limited to the one shown in Fig. 1. Rather, the mixing can take place anywhere within the blood treatment apparatus 100 or the tubing attached to it. In the example of Fig. 2, instead of feeding concentrates from sources A and B into a mixing apparatus 63 such as the one shown in Fig. 1, a fluid line into which the concentrates may be fed downstream the water source 55 is used as a mixing chamber.

During the first phase, the priming liquid contains no bicarbonate, a priming takes place with acid concentrate diluted in water only.

Water gained from a reverse-osmosis (RO) process or any other type of water such as tap water enters the mixing apparatus 63 from the water source 55.

Having passed an optional degassing device 60 the water may be heated in a heating apparatus 61 such that the temperature of the eventually generated priming liquid is between 25°C and 35°C (degrees Celsius).

Downstream of the heating apparatus 61 acid concentrate from source A is pumped into the water flow in the mixing apparatus 63.

As indicated by the symbol denoting the pump corresponding the source B of bicarbonate concentrate and the representation of the valve right downstream of the pump no bicarbonate is mixed into the thus generated water/acid solution.

A conductivity cell 163a may be used to monitor and control the [Na⁺] concentration of the priming liquid prepared in the mixing apparatus 63. In the first phase, a conductivity of 14.0 [mS] is measured.

A desirable [Na⁺] concentration is 137 mmol/l. As can be seen from Fig. 2, the [Na⁺] concentration of the priming liquid originates exclusively from the source A since no fluid and, hence, no sodium chloride or sodium [Na⁺] is contributed to the priming liquid from source B.

In the particular embodiment of Fig. 2, the container A comprises an acid concentrate for dialysis with sodium chloride in a concentration of 263 g/l NaCl. Further the container A comprises an acid, like citric acid or acetic acid, and preferably one or more of the following electrolytes, like magnesium chloride, calcium chloride and potassium chloride. For priming the extracorporeal circuit, the concentrate is diluted with water (33:1) to result in a priming solution comprising 137 mmol/l sodium.

**Fig. 3** shows a schematic representation of an exemplary implementation of the second phase of the method according to the present invention. The components shown in Fig. 3 are those of Fig. 2.

In the second phase bicarbonate concentrate is added to the priming liquid in preparation of the extracorporeal blood circuit 300 as is indicated by the icon representing the pump corresponding to the source B. In the particular setting shown in Fig. 3, the container A comprises 263 g/l NaCl. Its concentrate is used with a dilution factor 45:1 contributing 100 mmol/l sodium chloride, whereas the flow from container B contributes 37 mmol/l in an exemplary dilution having a dilution factor 25:1.

As in the first phase, the [Na⁺] concentration of the priming liquid is 137 mmol/l. However, the contribution from container A amounts to 100 mmol/l [Na⁺] and is, hence, less than before. In contrast to the first step, sodium chloride is contributed from the container B as well. In the example of Fig. 3, it also provides the lacking 37 mmol/l [Na⁺] needed for preparing a priming solution having a concentration of 137 mmol/l [Na⁺] as in the first phase.

The bicarbonate concentrate of container B may be saturated. In a saturated condition of the bicarbonate concentration of container B its sodium bicarbonate concentration may, in one exemplary embodiment, amount to 95,5 g/l at 20°C. Those number are, however, not intended to be limiting, of course.

It is noted that the priming solution prepared in the first phase, which is exemplary described with respect to Fig. 2, may be used when circulating a liquid through parts of the extracorporeal blood circuit 300 and/or of the hydraulic system while a patient is temporarily disconnected from the blood treatment apparatus 100. Also, a solution used for rinsing parts of the extracorporeal blood circuit 300 and/or of the hydraulic system between two patients that are consecutively treated with more or less the same equipment. Before connecting the next patient to said equipment, the solution prepared by the mixing apparatus 63 will have been mixed as set forth with respect to the second phase, e.g., as described supra.

### List of reference numerals

- 25: addition point for Heparin (optional)
- 29: venous blood chamber (optional)
- 31: de-aeration device
- 53: outlet
- 55: water source
- 57: heat exchanger
- 59: first flow pump
- 60: degassing device
- 61: heating apparatus
- 63: mixing apparatus
- 67: concentrate supply
- 69: concentrate supply
- 71: concentrate pump; sodium pump
- 100: blood treatment apparatus
- 101: blood pump
- 102: dialysate outlet line, effluent inlet line
- 104: dialysis liquid inlet line
- 105: substituate line
- 107: pre-dilution valve
- 107a: line
- 109: post-dilution valve
- 109a: line
- 111: pump for substituate
- 121: pump for dialysis liquid
- 131: pump for dialysate or effluent
- 150: control device or closed-loop control device

- 161: device
- 163a: conductivity sensor
- 163b: conductivity sensor
- 165a: temperature sensor
- 165b: temperature sensor
- 167: leakage sensor
- 169: second flow pump

- 300: extracorporeal blood circuit
- 301: first line (arterial line section)
- 302: first tubing clamp
- 303: blood filter or dialyzer
- 303a: dialysis liquid chamber
- 303b: blood chamber
- 303c: semi-permeable membrane
- 305: second line (venous line section)
- 306: (second) tubing clamp
- 315: detector
- 317: single-needle chamber
- 319: detector

- F1: filter
- F2: filter

- [Na⁺]: sodium concentration

- A: container (source of acid)
- B: container (source of bicarbonate)
- P: pressure measuring points
- PS1: arterial pressure sensor (optional)
- PS2: arterial pressure sensor (optional)
- PS3: pressure sensor (optional)
- PS4: pressure sensor for measuring the filtrate pressure (optional)
- V: valves
- VB: bypass valves
- Y: Y-connector

## Claims

1. A method of priming an extracorporeal blood circuit (300) using a blood treatment apparatus (100) comprising a dialysis liquid preparation system having a source (55) of water, a source (B) of bicarbonate concentrate and a source (A) of acid concentrate including sodium chloride, and configured to prepare a dialysis liquid from water, said bicarbonate concentrate and said acid concentrate only,
said extracorporeal blood circuit (300) comprises an arterial line section (301), connectable to a patient, for drawing blood from the patient, a venous line section (305), connectable to the patient, for returning the blood to the patient, and a blood side compartment or blood chamber (303b) of a blood filter (303), said method encompassing the steps of a first phase:
- preparing a priming solution from said source (55) of water and said source (A) of acid concentrate only to obtain an acid/water solution,
- connecting said arterial line section (301) to an outlet of the dialysis liquid preparation system of the blood treatment apparatus (100) and,
- filling the extracorporeal blood circuit (300) with said priming solution
**characterized in that**
- the dialysis liquid preparation system is not having, or is not being connected to, a source that comprises sodium chloride only or exclusively; and **in that**
- bicarbonate from the source (B) of bicarbonate concentrate is absent from said solution.

2. The method according to claim 1, wherein during the first phase the temperature of the priming solution is kept below 35°C, preferably between 25°C and 30°C.

3. The method according to any of the preceding claims, further encompassing the following step of a second phase, following the first phase, encompassing
- adding bicarbonate from the source (B) of bicarbonate concentrate to the priming solution.

4. The method according to claim 3, wherein during the second phase the temperature of the priming solution is raised to at least 35°C.

5. The method according to claim 3 or 4, wherein the priming and in particular the circulation of the priming solution within the extracorporeal circuit (300) is continued for at least 5 minutes before the priming is considered as completed.

6. A control device or closed-loop control device (150) configured to carry out or to initiate the method according to any one of the claims 1 to 5 in interaction with a blood treatment apparatus (100).

7. A blood treatment apparatus (100) for the extracorporeal treatment of a patient's blood, comprising
- a control device or closed-loop control device (150) according to claim 6.

8. The blood treatment apparatus (100) according to claim 7, wherein the blood treatment apparatus (100) is embodied as a hemodialysis apparatus, hemofiltration apparatus or hemodiafiltration apparatus, in particular as an apparatus for acute or chronic renal replacement therapy.

9. The blood treatment apparatus (100) according to any one of claims 7 to 8, with sensors (163a, 163b, 165a, 165b), arranged and configured in order to measure the sodium concentration ([Na⁺]), in the priming solution.

10. A digital storage medium, particularly in the form of a diskette, storage card, CD, DVD Blu-ray disc or (E)EPROM, with electronically readable control signals, configured to interact with a programmable control device or closed-loop control device, so that the steps of the method according to any one of the claims 1 to 5 are initiated, or so that a conventional control device or closed-loop control device is reprogrammed into a control device or closed-loop control device (150) according to claim 6.

11. A computer program product with a program code, saved on a machine-readable carrier, for initiating the steps of the method according to any one of the claims 1 to 5 when the computer program product is running on a control device or closed-loop control device, or to cause a conventional control device or closed-loop control device to be reprogrammed into a control device or closed-loop control device (150) according to claim 6.

12. A computer program with a program code for initiating the steps of the method according to any one of claims 1 to 5 if the computer program is running on a control device or closed-loop control device, or for causing a conventional control device or closed-loop control device to be reprogrammed into a control device or closed-loop control device (150) according to claim 6.

## Patentansprüche

1. Ein Verfahren zum Befüllen eines extrakorporalen Blutkreislaufs (300) unter Verwendung eines Blutbehandlungsgeräts (100), das ein Dialysatvorbereitungssystem umfasst, das eine Wasserquelle (55), eine Quelle (B) für Bicarbonatkonzentrat und eine Quelle (A) für Säurekonzentrat, einschließlich Natriumchlorid, aufweist und so konfiguriert ist, dass es Dialysat ausschließlich aus Wasser, dem Bicarbonatkonzentrat und dem Säurekonzentrat herstellt,
wobei der extrakorporale Blutkreislauf (300) einen arteriellen Leitungsabschnitt (301) umfasst, der mit einem Patienten verbunden werden kann, um Blut vom Patienten zu entnehmen, einen venösen Leitungsabschnitt (305), der mit dem Patienten verbunden werden kann, um das Blut dem Patienten zurückzuführen, und eine Blutseitekammer oder ein Blutkammer (303b) eines Blutfilters (303), wobei das Verfahren die Schritte einer ersten Phase umfasst:
- Herstellen einer Befülllösung ausschließlich aus der besagten Wasserquelle (55) und der besagten Quelle (A) des Säurekonzentrats, um eine Säure-Wasser-Lösung zu erhalten,
- Verbinden des arteriellen Leitungsabschnitts (301) mit einem Auslass des Dialysatvorbereitungssystems des Blutbehandlungsgeräts (100) und,
- Befüllen des extrakorporalen Blutkreislaufs (300) mit der besagten Befülllösung,
**dadurch gekennzeichnet, dass**
- das Dialysatvorbereitungssystem keine oder keine angeschlossene Quelle aufweist, die ausschließlich Natriumchlorid enthält; und dass
- Bicarbonat aus der Quelle (B) für Bicarbonatkonzentrat in der besagten Lösung fehlt.

2. Verfahren nach Anspruch 1, wobei während der ersten Phase die Temperatur der Befülllösung unter 35°C gehalten wird, vorzugsweise zwischen 25°C und 30°C.

3. Verfahren nach einem der vorhergehenden Ansprüche, das ferner den folgenden Schritt einer zweiten Phase umfasst, die auf die erste Phase folgt:
- Hinzufügen von Bicarbonat aus der Quelle (B) für Bicarbonatkonzentrat zur Befülllösung.

4. Verfahren nach Anspruch 3, wobei während der zweiten Phase die Temperatur der Befülllösung auf mindestens 35°C erhöht wird.

5. Verfahren nach Anspruch 3 oder 4, wobei das Befüllen und insbesondere die Zirkulation der Befülllösung innerhalb des extrakorporalen Kreislaufs (300) für mindestens 5 Minuten fortgesetzt wird, bevor das Befüllen als abgeschlossen gilt.

6. Eine Steuereinrichtung oder Regeleinrichtung (150), die konfiguriert ist, um das Verfahren gemäß einem der Ansprüche 1 bis 5 in Interaktion mit einem Blutbehandlungsgerät (100) auszuführen oder einzuleiten.

7. Ein Blutbehandlungsgerät (100) zur extrakorporalen Behandlung des Blutes eines Patienten, umfassend:
- eine Steuereinrichtung oder Regeleinrichtung (150) gemäß Anspruch 6.

8. Blutbehandlungsgerät (100) gemäß Anspruch 7, wobei das Blutbehandlungsgerät (100) als Hämodialysegerät, Hämofiltrationsgerät oder Hämodiafiltrationsgerät, insbesondere als Gerät für die akute oder chronische Nierenersatztherapie, ausgeführt ist.

9. Blutbehandlungsgerät (100) gemäß einem der Ansprüche 7 bis 8, mit Sensoren (163a, 163b, 165a, 165b), die so angeordnet und konfiguriert sind, dass sie die Natriumkonzentration ([Na+]) in der Befülllösung messen.

10. Ein digitales Speichermedium, insbesondere in Form einer Diskette, Speicherkarte, CD, DVD, Blu-ray Disc oder (E)EPROM, mit elektronisch lesbaren Steuersignalen, das so konfiguriert ist, dass es mit einer programmierbaren Steuereinrichtung oder Regeleinrichtung interagiert, sodass die Schritte des Verfahrens gemäß einem der Ansprüche 1 bis 5 eingeleitet werden oder dass eine konventionelle Steuereinrichtung oder Regeleinrichtung in eine Steuereinrichtung oder Regeleinrichtung (150) gemäß Anspruch 6 umprogrammiert wird.

11. Ein Computerprogrammprodukt mit einem Programmcodes, gespeichert auf einem maschinenlesbaren Träger, zur Einleitung der Schritte des Verfahrens gemäß einem der Ansprüche 1 bis 5, wenn das Computerprogrammprodukt auf einer Steuereinrichtung oder Regeleinrichtung läuft, oder um eine konventionelle Steuereinrichtung oder Regeleinrichtung in eine Steuereinrichtung oder Regeleinrichtung (150) gemäß Anspruch 6 umzuprogrammieren.

12. Ein Computerprogramm mit einem Programmcodes zur Einleitung der Schritte des Verfahrens gemäß einem der Ansprüche 1 bis 5, wenn das Computerprogramm auf einer Steuereinrichtung oder Regeleinrichtung läuft, oder um eine konventionelle Steuereinrichtung oder Regeleinrichtung in eine Steuereinrichtung oder Regeleinrichtung (150) gemäß Anspruch 6 umzuprogrammieren.

## Revendications

1. Un procédé d'amorçage d'un circuit sanguin extracorporel (300) utilisant un appareil de traitement du sang (100) qui comprend un système de préparation de liquide de dialyse ayant une source (55) d'eau, une source (B) de concentré de bicarbonate ainsi qu'une source (A) de concentré acide incluant du chlorure de sodium, et configuré pour préparer un liquide de dialyse uniquement à partir d'eau, dudit concentré de bicarbonate et dudit concentré acide,
ledit circuit sanguin extracorporel (300) comprend une section de conduite artérielle (301), raccordable à un patient, pour prélever le sang du patient, une section de conduite veineuse (305), raccordable au patient, pour renvoyer le sang au patient, ainsi qu'un compartiment côté sang ou une chambre à sang (303b) d'un filtre sanguin (303), ledit procédé englobant les étapes d'une première phase consistant à :
- préparer une solution d'amorçage uniquement à partir de ladite source (55) d'eau et de ladite source (A) de concentré acide, pour obtenir une solution acide/eau,
- raccorder ladite section de conduite artérielle (301) à une sortie du système de préparation du liquide de dialyse de l'appareil de traitement du sang (100) et,
- remplir le circuit sanguin extracorporel (300) avec ladite solution d'amorçage.
**caractérisé en ce que** :
- le système de préparation du liquide de dialyse ne possède pas, ou n'est pas raccordé à, une source qui comprend uniquement ou exclusivement du chlorure de sodium;
et **en ce que** :
- le bicarbonate provenant de la source (B) de concentré de bicarbonate est absent de ladite solution.

2. Le procédé selon la première revendication, où, pendant la première phase, la température de la solution d'amorçage est maintenue en dessous de 35°C, de préférence entre 25°C et 30°C.

3. Le procédé selon l'une quelconque des revendications précédentes, englobant en outre l'étape suivante d'une seconde phase, suivant la première phase, consistant à :
- ajouter du bicarbonate provenant de la source (B) de concentré de bicarbonate à la solution d'amorçage.

4. Le procédé selon la revendication 3, où pendant la seconde phase, la température de la solution d'amorçage est élevée à au moins 35°C.

5. Le procédé selon la revendication 3 ou 4, où l'amorçage et notamment la circulation de la solution d'amorçage dans le circuit extracorporel (300) se poursuit pendant au moins 5 minutes avant que l'amorçage ne soit considéré comme terminé.

6. Un dispositif de commande ou un dispositif de commande en boucle fermée (150), configuré pour exécuter ou pour initier le procédé selon l'une quelconque des revendications 1 à 5, en interaction avec un appareil de traitement du sang (100).

7. Un appareil de traitement du sang (100) pour le traitement extracorporel du sang d'un patient, comprenant :
- un dispositif de commande ou un dispositif de commande en boucle fermée (150), selon la revendication 6.

8. L'appareil de traitement du sang (100) selon la revendication 7, où l'appareil de traitement du sang (100) est conçu sous la forme d'un appareil d'hémodialyse, d'un appareil d'hémofiltration ou d'un appareil d'hémodiafiltration, notamment sous la forme d'un appareil pour la thérapie de remplacement rénal aiguë ou chronique.

9. L'appareil de traitement du sang (100) selon l'une quelconque des revendications 7 à 8, avec des capteurs (163a, 163b, 165a, 165b), agencés et configurés pour mesurer la concentration de sodium ([Na⁺]) dans la solution d'amorçage.

10. Un support de stockage numérique, notamment sous la forme d'une disquette, d'une carte mémoire, d'un CD, d'un DVD, d'un disque Blu-ray ou d'une EPROM (E), avec des signaux de commande lisibles électroniquement, configuré pour interagir avec un dispositif de commande programmable ou un dispositif de commande en boucle fermée, de sorte que les étapes du procédé selon l'une quelconque des revendications 1 à 5 soient initiées, ou qu'un dispositif de commande conventionnel ou un dispositif de commande en boucle fermée soit reprogrammé en un dispositif de commande ou un dispositif de commande en boucle fermée (150) selon la revendication 6.

11. Un produit de programme informatique comportant un code de programme, enregistré sur un support lisible par machine, pour initier les étapes du procédé selon l'une quelconque des revendications 1 à 5 lorsque le produit de programme informatique est exécuté sur un dispositif de commande ou sur un dispositif de commande en boucle fermée, ou pour engendrer la reprogrammation d'un dispositif de commande conventionnel ou d'un dispositif de commande en boucle fermée en un dispositif de commande ou un dispositif de commande en boucle fermée (150) selon la revendication 6.

12. Un programme informatique comportant un code de programme pour initier les étapes du procédé selon l'une quelconque des revendications 1 à 5 si le programme informatique est exécuté sur un dispositif de commande ou sur un dispositif de commande en boucle fermée, ou pour engendrer la reprogrammation d'un dispositif de commande conventionnel ou d'un dispositif de commande en boucle fermée en un dispositif de commande ou un dispositif de commande en boucle fermée (150) selon la revendication 6.
